# FASCICULE DE BREVET EUROPEEN

(11) **EP 0 523 228 B1**
(45) Date de publication et mention de la délivrance du brevet: **12.08.1998**
(21) Numéro de dépôt: 92906391.5
(22) Date de dépôt: 05.02.1992
(51) Int. Cl.: A61B 5/16

(54) **REALISATION DE TESTS PSYCHOMETRIQUES ET COGNITIFS ET ENREGISTREMENT DES RESULTATS**
VERFAHREN UND VORRICHTUNG ZUR REALISIERUNG VON KOGNITIVEN UND PSYCHOMETRISCHEN PRÜFUNGEN UND AUFZEICHNUNG VON ERGEBNISSEN
IMPLEMENTATION OF PSYCHOMETRIC AND COGNITIVE TESTS AND RECORDING OF RESULTS

(30) Priorité: 06.02.1991 FR 9101700
(43) Date de publication de la demande: 20.01.1993
(73) Titulaire: BIOPHYDERM S.A., 34940 Montpellier cedex 9 (FR)
(72) Inventeur: Nouguier, Jean, 34270 Valflaunes (FR); Nouguier-Soulé, Jeanine, 34270 Valflaunes (FR); Reinberg, Alain, 75016 Paris (FR)
(86) Numéro de dépôt international: FR9200103
(87) Numéro de publication internationale: WO9213487

(56) Documents cités:
- EP-A- 251 541
- DE-A- 2 947 536
- DE-A- 3 703 404
- US-A- 4 285 517
- US-A- 4 755 140

## Description

La présente invention concerne un procédé et un dispositif programmable, miniaturisé, autonome et ambulatoire, destinés à faire effectuer par un sujet humain des tests psychométriques, à en mesurer et enregistrer les temps de réponse et les résultats, dans le but d'apprécier l'évolution chronologique des performances et du niveau de vigilance dudit sujet.
Le domaine technique de l'invention est celui des dispositifs de mesure.
Les tests psychométriques et notamment la mesure du temps de réaction sont utilisés en psychologie et psychiatrie pour évaluer les capacités attentionnelle et mnésique de sujets dans le cadre d'explorations à visée clinique ou dans le cadre de la surveillance de sujets pour lesquels la vigilance présente une importance particulière comme les travailleurs postés ou les sportifs de haut niveau.
On connaît un certain nombre de dispositifs qui assurent soit la mesure du Temps de Réaction ou TR, soit l'évaluation des réponses à des épreuves de vigilance ou d'attention, soit la délivrance de stimuli en vue de l'entraînement sportif. Les mesures sont généralement effectuées à partir de postes fixes et sous la direction d'un opérateur, ou dans certains cas à l'aide d'un micro-ordinateur.
La demande N° EP-A-0 251 541 décrit un dispositif pour accroître et développer la performance neuromusculaire réflexe sous des conditions contrôlées qui permettent l'entrainement aux sports de combat. Il s'agit de réagir aussi vite que possible à un stimulus lumineux ou sonore par un coup frappé sur une ou plusieurs cibles avec une force qui est évaluée. La vitesse de la réponse est utilisée pour l'entraînement du sujet.
La demande N° DE-A-3 703 404 décrit un dispositif réalisant par l'intermédiaire d'un système d'affichage et de boutons, le remplissage de questionnaires et d'échelles analogiques normalisés et couramment utilisés en milieu hospitalier, qui permettent d'évaluer des paramètres codifiés concernant l' humeur, la qualité du sommeil ou l'intensité de la souffrance du sujet, remplissage classiquement effectué sur papier. Ce dispositif indique par un signal sonore le début de lu séquence de questions auxquelles le sujet répond en positionnant éventuellement un curseur sur une échelle analogique, le résultat, qui est la position du curseur sur la ligne, étant acquis lorsque ledit curseur n'a pas bougé pendant 2 minutes.
La demande N° FR2 444 452 décrit un dispositif de mesure de temps de réaction simple, temps de réaction sélectif, temps de réaction des excitations communes et temps de réaction par rapport au début ou à la cessation de l'excitation lumineuse ou phonique. C'est un dispositif de table utilisant une imprimante comme sortie de résultats durant l'exploration fonctionnelle du sujet.
Ces dispositifs connus concernent principalement le temps de réaction et la réponse à des questionnaires standards en psychologie.
Or, indépendamment de son intérêt pour les recherches en chronobiologie, la connaissance des variations circadiennes, c'est à dire de période égale à ou peu différente de 24h, ou ultradiennes, c'est à dire de période inférieure à 24h, ou infradiennes, c'est à dire de période supérieure à 24h, du TR et de la vigilance d'un sujet présente un intérêt certain dans la mesure où ces paramètres, corrélés avec d'autres données de l'organisme peuvent avoir, chez l'individu sain comme chez le malade, une valeur représentative de son status psychophysiologique.
De nombreuses études ont mis en évidence le fait qui différentes performances mentales, psychomotrices et cognitives, telles que celles obtenues avec le temps de réaction et des épreuves dites de vigilance, varient au cours de la journée en même temps que certaines variables physiologiques. L'efficience dans ces épreuves est mesurée à partir du nombre d'éléments correctement détectés, du nombre d'erreurs effectuées, et du temps écoulé entre l'apparition du signal et la production de la réponse. Les secteurs de l'activité humaine principalement concernés par les conséquences de ces fluctuations sont le travail, la santé, l'éducation et le sport.
Le temps de réaction TR peut se définir comme la durée que met un sujet pour réagir volontairement, par un mouvement convenu, à une stimulation sensorielle par exemple auditive ou visuelle, plus ou moins complexe. On distingue le temps de réaction simple ou temps de réponse d'un sujet à un événement annoncé, qui survient un certain temps après son annonce, et le temps de réaction de choix peut être défini comme le temps de réponse à un évènement réclamant une discrimination de la part du sujet au moment où il survient. Par exemple si la condition imposée est " lumière rouge réponse a droite, lumière verte réponse à gauche, lumière jaune réponse des deux mains", lorsque un stimulus lumineux de couleur rouge survient, le sujet doit déterminer la modalité de réponse; dans ce cas, des épreuves d'apprentissage et d'essai sont prévues avant le test proprement dit. Le temps de réaction dépend de nombreux facteurs à la fois physiologiques, psychologiques, environnementaux, et liés a la nature de la tâche; il est considéré comme un indicateur pertinent dans l'évaluation de nombreux processus sensoriels et mentaux.

Des explorations plus complètes nécessitent l'utilisation de batteries de tests comportant généralement des épreuves de barrage de lettres, de calcul mental, de scrutage mnésique, de mémorisation spaciale, de pistage instable, de raisonnement logique et de tâche double. Une épreuve de barrage de lettres consiste à demander au sujet de mémoriser un ou plusieurs signes qui peuvent être des lettres de l'alphabet. On lui présente ensuite une succession de pages composées d'un certain nombre de signes parmi lesquels il doit cocher ceux qu'on lui a demandé de mémoriser.
Pour une épreuve de calcul mental on demande au sujet d'effectuer des successions d'opérations simples prises parmi l'addition et/ou la soustraction et/ou la multiplication et/ou la division appliquées à des nombres successifs de 1 ou plusieurs chiffres.
Un test de scrutage mnésique peut consister à demander au sujet de mémoriser un ou plusieurs mots ou items tests puis à lui présenter pendant un temps donné un ou plusieurs ensembles de mots ou items, designés comme cibles. Lorsque la cible disparaît, le sujet doit dire si elle contenait le ou les items tests.
Un test de mémorisation spaciale peut consister à présenter au sujet une figure géométrique ,un histogramme par exemple, pendant quelques secondes, puis à lui présenter une autre figure différant ou non de la première et ayant subi par exemple une rotation à gauche ou à droite. On demande si l'enveloppe de la 2ième figure est la même que celle de la première.
Un test de pistage instable permet de mesurer la capacité du sujet à maintenir un pointeur près d'une cible, sous certaines contraintes imposées aux trajectoires du curseur et de la cible.
Un test de raisonnement logique peut être réalisé de la façon suivante: on présente au sujet une proposition logique telle que "A et C précèdent B" qu'il doit mémoriser. On présente ensuite une combinaison des items mis en jeu dans la proposition logique par exemple "A B C". Le sujet doit répondre par VRAI ou FAUX.
Un test de tâche double peut être effectué en présentant par exemple un scrutage mnésique à un sujet en train d'effectuer un pistage instable, la réponse au scrutage mnésique étant donnée par exemple par le biais d'un interrupteur à pédale. L'exactitude des réponses et la vitesse d'exécution de ces tests permettent d'apprécier les capacités attentionnelles et mnésiques (mémoire à court terme et à long terme) du sujet auquel ils sont appliqués. La réalisation de ces tests de manière répétitive permet de cerner les variations de son activité intellectuelle par la mesure des fluctuations de son efficience dans la réalisation de tâches de complexités variées. La mesure liée à ces tests est composite puisque elle peut être constituée selon le cas et de manière non exclusive d'un temps de réponse à une demande, d'un score relatif à une épreuve, et d'une information sur la qualité de la réponse. Il faut ajouter à ces tests l'utilisation par un sujet d'echelles d'auto-estimation, de la vigilance par exemple et, éventuellement la mesure de paramètres physiques, par exemple la force musculaire.

Un problème posé qui n'est pas résolu par les procédés et dispositifs connus est de procurer un procédé et un dispositif permettant la réalisation de l'ensemble des tests cognitifs ci- dessus décrits et l'enregistrement de leurs résultats pendant plusieurs semaines; le problème posé est également de permettre au sujet de réaliser ces tests de façon autonome au cours de son activité journalière, dans son environnement habituel, à son domicile et sur ses lieux de travail et de loisirs. Le problème est également de fournir un procédé et un dispositif qui permette de faire varier les paramètres des tests , pour mieux les adapter aux sujets auxquels ils sont appliqués, par une programmation à l'aide d'un ordinateur central, au cours d'une liaison temporaire; le problème est aussi de fournir un dispositif qui soit de poids et de dimensions compatibles avec un port quasi permanent par le sujet.

L'invention a pour but de proposer une solution aux problèmes posés.

Une solution au problème posé consiste à procurer un procédé pour la réalisation de tests de temps de réaction simple et de choix, de barrage de lettres, de calcul mental, de scrutage mnésique, de raisonnement logique, de mémorisation spaciale, de pistage instable, et de tâches doubles , pour la création d'échelles d'autoestimation et la mesure de la force musculaire, et pour l'enregistrement et l'analyse des résultats au moyen d' un ensemble compact portable disposant d'une autonomie d'énergie suffisante pour alimenter durant plusieurs semaines le dispositif de commande qui délivre des stimuli par le biais de transducteurs acoustiques et optiques, gère les moyens d'affichage et de synthèse vocale et toutes les transactions nécessaires à la réalisation des tests , se caractérise en ce qu'il comporte les opérations ci-dessous décrites :
- on procure un dispositif portable compact qui comporte lesdits transducteurs acoustique et optique, lesdits moyens d'affichage, ledit dispositif de synthèse vocale, ledit dispositif de commande, ladite réserve d'énergie, lequel dispositif comporte au moins un micro- processeur qui exécute un programme d'instructions stockées dans une première mémoire , lequel dispositif comporte au moins une deuxième mémoire, une horloge, un dispositif de comptage d'événements, un dispositif de mesure de paramètres , des films résistifs de détection de force et de position, des interrupteurs à contact temporaire, lequel dispositif comporte une interface bi-directionnelle de communication,
   On enregistre dans la première mémoire PROM ledit programme,
- on procure au moins un ordinateur central, qui peut être un serveur, et des moyens de communication par voie directe et par voie télématique entre le dit ordinateur central ou le serveur, et le dit dispositif portable,
- on enregistre durant une première phase de communication, dans la dite seconde mémoire RAM les paramètres qui ont été fixés en fonction du sujet et de l'objectif de l'enregistrement à l'aide de logiciele spécifiques s'exécutant sur l'ordinateur central ou la serveur, paramètres selon lesquels les stimuli seront émis et les réponses du sujet seront demandées et stockées, c'est à dire par exemple le nombre d'échelles d'auto-estimation qui seront proposées, les intitulés des échelles libres, le nombre d'épreuves de TR simple, le nombre de séquences d'apprentissage, d'essais et de tests pour le TR de chois, le nombre d'écrans proposés pour le calcul mental et d'items à mémoriser pour le barrage de lettres, le nombre de chiffres et la nature des opérations proposées pour le calcul mental, par exemple pour des tests destinés à des enfants de classes élémentaires proposer des séries d'additions et de soustractions sur des nombres <=20 tirés au sort, mais de façon que le résultat soit toujours positif, lesquels paramètres seront aussi par exemple le nombre d'items tests et le nombre de cibles pour le scrutage mnésique, le nombre d'histogrammes présentés pour la mémorisation spaciale, le temps pendant lequel le pistage instable est proposé, le nombre d'items impliqués dans la proposition logique pour le test de raisonnement logique, les tâches proposées pour le test de tâche double, le nombre d'épreuves de force musculaire, et pour chaque test l'affichage ou non des scores,
- et dans une phase de fonctionnement autonome et ambulatoire, conformément au dit programme et aux dits paramètres, le sujet reçoit pour chaque épreuve, par l'intermédiaire soit du dispositif d'affichage à cristaux liquides, soit du dispositif de synthèse vocale , par le biais du transducteur acoustique intégré ou des écouteurs annexes, des directives concernant d'une part la nature du test ou des stimuli qui vont lui être proposés d'autre part les réactions devant y être associées; les intitulés des échelles d'autoestimation concernant l'humeur et la qualité du sommeil sont proposés par des messages affichés ou vocaux; l' échelle sur laquelle le sujet porte son évaluation est constituée soit par une ligne tracée sur l'afficheur sur laquelle il déplace un curseur à l'aide des poussoirs gauche et droit, la position choisie étant validée par un appui simultané des deux poussoirs, soit par un film résistif de détection de position fixé sur le boîtier et sur lequel le sujet déplace une pointe rigide; le sujet indique une fois par jour, par le biais de défilements successifs de chiffres, ses heures de coucher, d'endormissement, de dernier éveil et de lever et remplit des échelles d'autoestimation de qualité du sommeil; les épreuves de tests et les stimuli sont réalisés grâce à des ressources optiques comprenant le dispositif d'affichage et au moins trois diodes électroluminescentes, des ressources sonores comprenant au moins trois fréquences émises par le transducteur acoustique et des ressources particulières telles que des films résistifs de détection de force; un générateur de nombres aléatoires permet de tirer au hasard les items et les latences lorsque les tests décrits l'imposent; un ensemble de moyens de comptage d'évènements, de durées et de fréquences, pour la mesure de la fréquence de l'oscillateur commandé par le film résistif capteur de force musculaire, sont activés; la mesure est constituée, selon le cas et de façon non exclusive, soit par le score obtenu, soit par le temps ne réponse du sujet aux tests et stimuli réalisés soit par l'index de conformité de la réponse à la demande, ladite mesure faisant l'objet d'un codage en vue de son analyse statistique ultérieure, par exemple pour le test de barrage de lettres la mesure est constituée du nombre exact d'items dans l'afficheur, du nombre d' items cochés, du nombre d'erreurs et du nombre d'items cochés plusieurs fois ce qui donne par différence le nombre d'oublis et permet d' analyser finement les types d'erreurs et pour le temps de réaction, la mesure inclut un code indiquant si la réponse a été faite à droite, à gauche ou des 2 mains et si il y a eu réponse anticipée, ce qui permet de déceler les effets de latéralisation préférentielles,
- et on conserve les résultats dans la mémoire RAM, selon un des dits paramètres, ou bien sous la forme de mesures discrètes ou bien sous une forme statistique consistant soit en la moyenne et l'écart-type d'un nombre de mesures paramétré, soit en un histogramme des fréquences des mesures pour chaque modalité du test, avec un nombre de classes également paramétré,
- et dans toute autre phase de communication, tant que l' initialisation du dispositif n'a pas été effectuée, on lit dans la mémoire RAM, puis on transfère dans le dit ordinateur central les dites valeurs des dits temps de réaction, la capacité d'enregistrement étant d'au moins 60000 mesures, la durée d'autonomie étant fonction de la fréquence journalière des tests soit environ 25 jours pour 8 prises de données par jour réalisant chacune 10 échelles d'autoestimation, 3 épreuves de calcul mental, la présentation de 6 pages de 64 lettres pour le barrage de lettres, 30 épreuves de TR simple et 30 de TR de choix, 3 épreuves de scrutage mnésique, 3 épreuves de raisonnement logique et quelques mesures de force musculaire.

Une solution au problème posé consiste également à procurer un dispositif autonome miniaturisé portable par un sujet humain, caractérisé en ce qu'il comprend:
- au moins 2 interrupteurs a contact temporaire permettant au sujet de répondre au stimulus soit de la main droite, soit de la main gauche,
- au moins 1 prise permettant la connection d'accessoires tels qu' interrupteurs pédale,
- au moins un transducteur acoustique qui confère au dispositif la possibilité d'émettre des sons de diverses fréquences ainsi que de délivrer des messages par synthèse vocale,
- au moins un transducteur optique qui confère au dispositif la capacité d'émettre des signaux lumineux et qui peut avantageusement comprendre une ou plusieurs diodes électro-luminescentes de couleurs différentes par exemple rouge, verte et jaune,
- un dispositif de commande constitué par un module électronique comportant au moins un microprocesseur associé à des moyens de mémorisation constitués d'au moins une première mémoire PROM renfermant sous forme de programme les instructions pour le fonctionnement de base du dit dispositif et pour le traitement statistique des données, d'au moins une seconde mémoire RAM dans laquelle sont transférés les paramètres nécessaires au fonctionnement programmé et autonome du dit dispositif et enregistrés les résultats des tests,
- au moins une horloge comportant une base de temps, pilotée par quartz,
- au moins un dispositif de mesure du temps de réponse constitué par un ensemble de comptage mesurant le temps d'ouverture des interrupteurs entre le début du stimulus et leur fermeture par le sujet,
- au moins un dispositif de comptage d'évènements,
- au moins un dispositif de synthèse vocale associé à une troisième mémoire PROM et à la mémoire RAM renfermant les données nécessaires a la synthèse d'un certain nombre de mots prononcés par le dispositif à l'intention du sujet,
- au moins un microphone qui peut avantageusement être un microphone à électrets , destiné à détecter les réponses du sujet par "OUI" et "NON",
- un dispositif d'affichage comportant au moins quatre lignes d'au moins seize caractères alphanumériques composés par matrice de points, et pouvant être un afficheur graphique,
- au moins un film résistif de détection de force et au moins un film résistif de détection de position,
- au moins un dispositif d'interface bidirectionnel de communication,
- au moins une prise pour écouteurs.

En définitive, grâce aux dispositions conformes à l'invention il est possible de réaliser des suivis de l'évolution temporelle des performances mentales et psychomotrices d'un sujet, de manière ambulatoire et autonome, par la mise en oeuvre du procédé ci-dessus décrit, le sujet réalisant les tests au moyen du dispositif miniaturisé portable et autonome préalablement et dûment programmé lors de la première phase de connection à l'ordinateur central.

L'évolution de la technologie électronique autorise la réalisation du dit dispositif sous une forme très compacte avec de très faibles besoins énergétiques autorisant de lui incorporer une source électrique autonome telle que pile ou batterie d'accumulateurs de faible dimension. Il en résulte que le dispositif de faible poids et encombrement peut être transporté et gardé sur lui par le sujet sans géne particulière ce qui permet l'exécution des séquences de test aux moments les plus propices. L'invention sera mieux comprise à la lecture de la description qui suit d'un mode de réalisation préféré donné uniquement à titre d'exemple non limitatif.
- la figure 1 est une vue d'ensemble très shématisée des éléments mis en jeu dans le déroulement du procédé conformément à l'invention.
- la figure 2 représente le dispositif miniaturisé autonome conditionné dans un boitier.
- la figure 3 représente un organigramme de programmation du dispositif miniaturisé autonome en vue de son fonctionnement autonome et ambulatoire.
- la figure 4 représente un organigramme de l'ensemble des opérations réalisées par le dispositif.

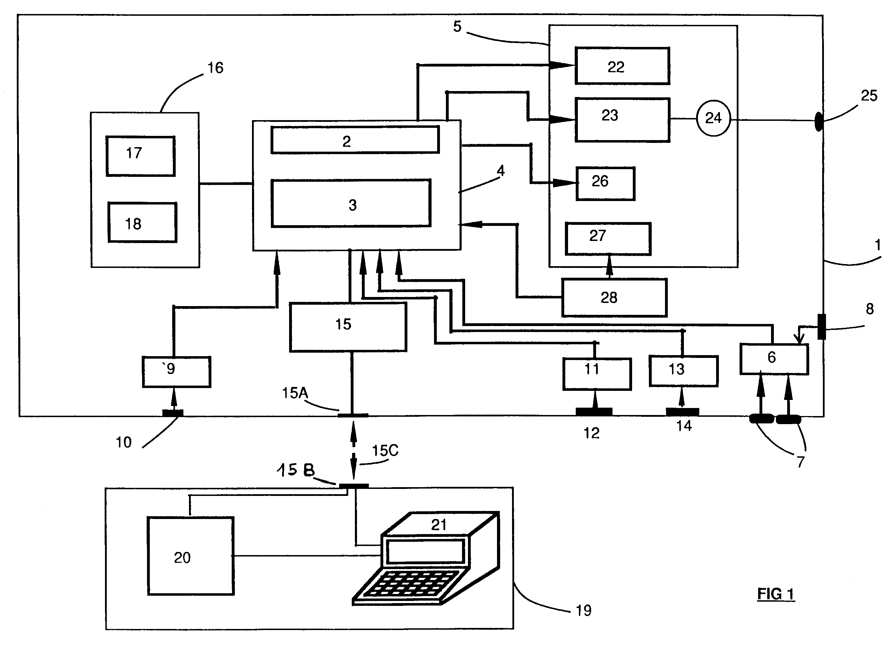

En se référant à la figure 1, le dispositif de mesure désigné dans son ensemble par la référence numérique 1 est agencé autour des unités de commande 2 et de gestion des informations 3, qui peuvent avantageusement être regroupées dans un circuit unique de type microprocesseur 4 qui commande un bloc de sortie 5, analyse les signaux d'entrée 6 résultant de la fermeture d'interrupteurs intégrés au boitier 7 et externes 8, analyse les signaux d'entrée 9 en provenance du microphone 10 et les signaux d'entrée 11 en provenance d'un film résistif de détection de forces 12 et les signaux de films résistifs de détection de position, lequel microprocesseur gère les informations transitant sur un interface 15 comportant un connecteur 15A pouvant coopérer avec un connecteur complémentaire 15B, et échange des informations avec le bloc de mémorisation 16, contenant les instructions nécessaires au fonctionnement du dispositif et auquel il est relié en permanence.

Le bloc de mémorisation 16 comprend une mémoire RAM 17 qui renferme d'une part les données transmises au cours de la phase de communication c'est à dire les caractéristiques des tests qui seront appliqués au sujet, y compris les données de synthèse vocale libres, et les caractéristiques du type de traitement qui sera appliqué aux données avant leur stockage dans la dite mémoire RAM 17. Ladite mémoire RAM 17 renferme d'autre part les données recueillies, sous la forme de flots ou de blocs d'octets. Par ailleurs le bloc de mémorisation 16 comprend une mémoire PROM 18 contenant des instructions permanentes nécessaires au fonctionnement du microprocesseur 4 et une troisième mémoire PROM (non représentée) renfermant les données nécessaires à la synthese d'un certain nombre de mots qui seront assemblés en phrases selon les instructions contenues dans la mémoire PROM 18 et les paramètres entrés dans la mémoire RAM 17.

Le microprocesseur 4 peut également être relié temporairement grâce à des moyens de communication 15C à un système informatique 19 auquel est associé un logiciel d'assistance, pour l'étude personnalisée des résultats, et de dialogue, pour l'introduction par l'opérateur dans la mémoire RAM 11 de toutes les données susmentionnées, lequel système informatique 19 peut être un système télématique 20, tel qu'un serveur auquel on accède par un modem et par un Minitel , et peut être aussi un ordinateur central 21 auquel le dispositif accède directement. La liaison entre le microprocesseur 4 et le système informatique 19 s'effectue par l'intermédiaire de l' unité d'interface 15 installée à demeure dans le dispositif 1, et fournissant par exemple des signaux électriques au standard RS232C.

Le bloc de sortie 5 est composé d'un afficheur 22, d'un système délivrant des phrases par synthèse vocale 23 et actionnant un transducteur acoustique avantageusement constitué par un haut parleur électromagnétique 24 de petites dimensions et une prise pour écouteur 25 connectée en parallèle sur le dit transducteur, d'un bloc de diodes électroluminescentes 26 avantageusement rouges vertes et jaunes et d'un avertisseur sonore 27 affecté à un signal d'alarme du circuit chien de garde 28 contrôlant le bon fonctionnement de base du microprocesseur.

Enfin l'alimentation électrique du dispositif 1 est réalisée par une source autonome (non représentée) telle que piles ou batterie ou analogue qui donne la possibilité de rendre l'ensemble plus compact et plus léger. Une alarme est prévue pour annoncer toute anomalie d'alimentation électrique ou une panne de fonctionnement.

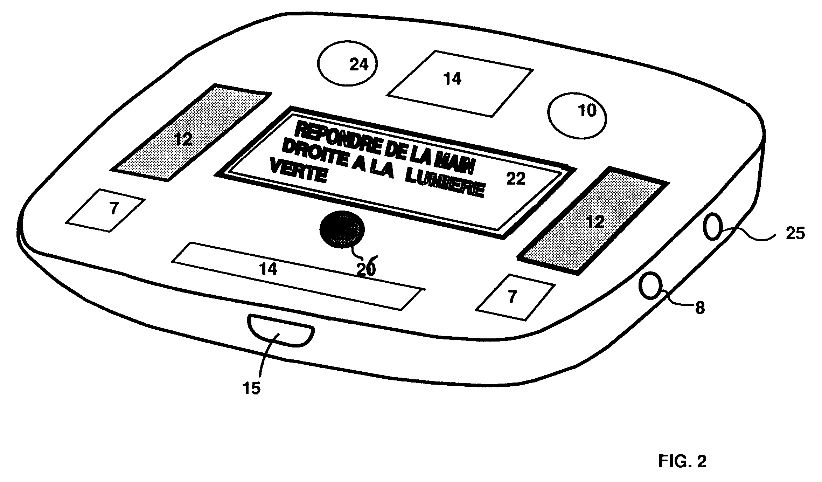

La figure 2 représente un dessin d'une réalisation préférée de ce dispositif, les dimensions du boitier de conditionnement étant inférieures à 9X13X4 cm.

Le mode de fonctionnement général peut être le suivant:

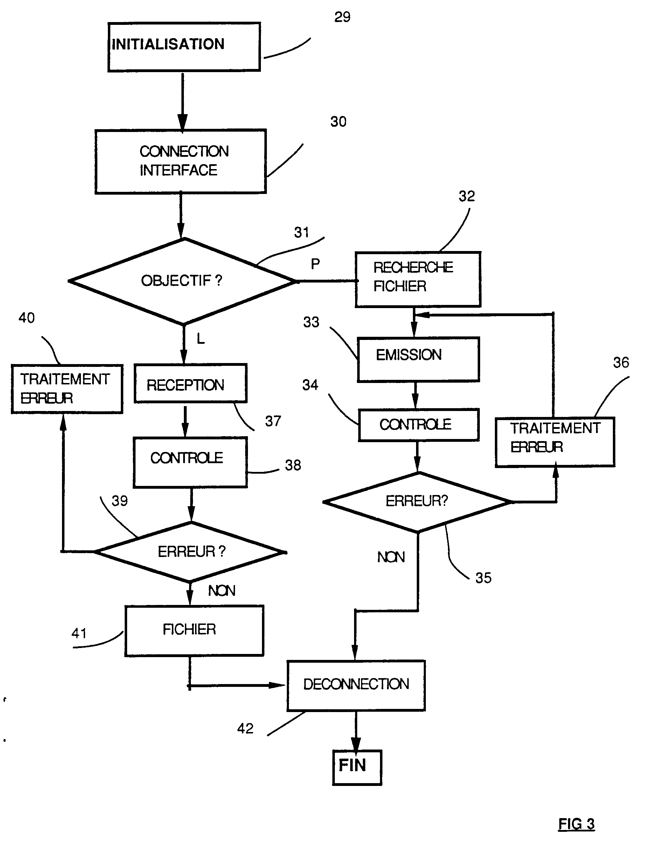

Le dispositif 1 doit tout d'abord être programmé pour assurer le déroulement des séquences de tests de la façon requise par le responsable de la programmation. Dans cette première phase le dispositif est connecté au système informatique (fonctionnement en mode connecté). En se reportant à l'organigramme représenté à la figure 3, après initialisation (bloc 29) l'opérateur connecte le dispositif autonome au système informatique (bloc 30) et opère différemment selon l'objectif de la connection (bloc 31); s'il désire effectuer une programmation, il constitue ou recherche le fichier des caractéristiques des tests et prépare la mise à l'heure du dispositif (bloc 32). L'information est ensuite transférée vers le dispositif via l'interface RS232 dans la RAM 17 (bloc 33). Un contrôle de parité et de somme de contrôle avec retenue est effectué (Bloc 34); si le test d' erreur (bloc 35) est positif une procédure de traitement d'erreur est exécutée; si l'opérateur désire simplement lire les informations contenues dans la mémoire RAM du dispositif (bloc 37), le système informatique recoit les octets en provenance de cette mémoire via l'interface RS232C, un contrôle de parité et de somme de contrôle avec retenue est effectué (bloc 38); si le test d' erreur (bloc 39) est positif la procédure de traitement d'erreur est exécutée; dans le cas où la réception est bonne, les fichiers de résultats sont constitués; l'opération de dialogue se termine par la déconnection (bloc 42).

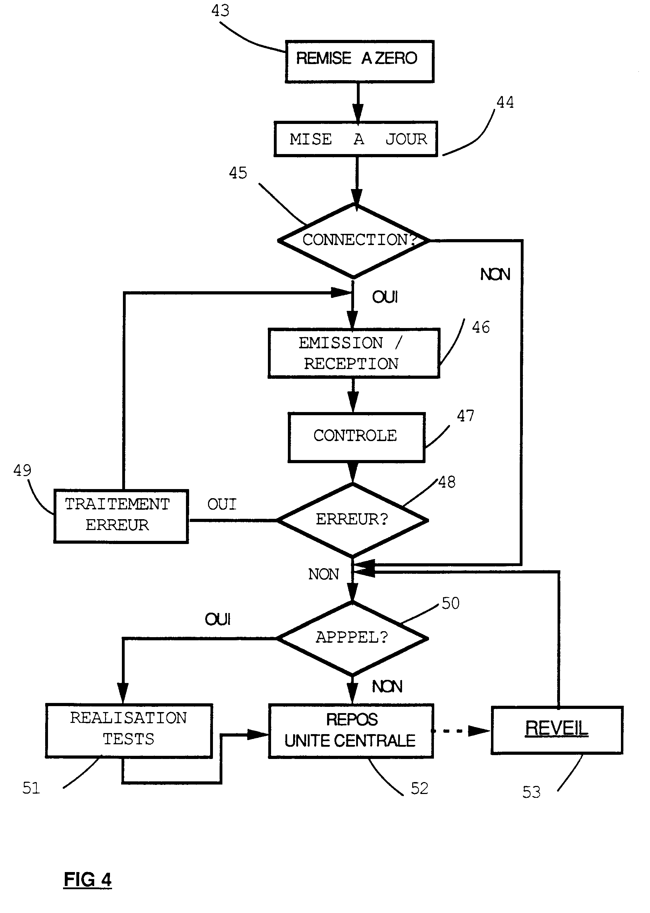

Après programmation et déconnection, le dispositif 1 est en mesure de fonctionner de manière autonome à partir des informations contenues dans le bloc de mémorisation 16, c'est à dire les informations générales de fonctionnement du dispositif contenues en permanences dans la mémoire PROM 18 et les informations propres au sujet et à l'enregistrement actuel, introduites dans la mémoire RAM 17. L'organigramme de la figure 4 auquel on se reporte maintenant illustre la fonction assurée par la mémoire PROM 18, qui renferme toutes les instructions nécessaires au fonctionnement du microprocesseur. Au moment où on alimente électriquement le dispositif autonome, une remise à 0 est effectuée (bloc 43) et des valeurs de fonctionnement sont affectées aux composants du dispositif (bloc 44).

En cas d'établissement d'un dialogue (bloc 45) entre le dispositif 1 et le système informatique, le microprocesseur assure s'il y a lieu la réception des paramètres selon lesquels les tests et stimuli seront délivrés et les résultats stockés, et assure aussi l'émission des données en vue de la constitution des fichiers de résultats sur le système informatique (bloc 46), il effectue un contrôle de parité et de somme de contrôle avec retenue (bloc 47), et si le test d'erreur (bloc 48) est positif, ledit microprocesseur réalise la procédure de traitement d'erreur (bloc 49).

En l'absence de dialogue, et après programmation le microprocesseur 4 a la possibilité d'identifier les demandes d' entrée en communication avec le sujet (bloc 50), qui surviennent soit par une requête programmée, par exemple un signal sonore toutes les 2 heures entre 8h et 22h, soit par une combinaison particulière de pressions sur les interrupteurs effectuée par le sujet, par exemple la pression simultanée des poussoirs droit et gauche reconnue par le dispositif et répétée sur demande dudit dispositif affichée ou orale. Il assure alors la présentation des échelles analogiques et la réalisation des tests (bloc 51). Pour chaque échelle analogique, il présente l'intitulé de la demande, active l'échelle sur laquelle le sujet va porter son appréciation, à partir d'une position initiale programmable du curseur s'il s'agit de l'échelle de l'afficheur, et attend que le sujet valide une position sur le support prévu. Cette position est conservée en pourcentage de la longueur totale de l'échelle. Les informations concernant le sommeil sont données une fois par jour; elles comportent des échelles d'autoestimation de qualité et l'indication de heure de coucher, d'endormissement, de dernier éveil et de lever, entrées par le sujet par défilement grâce aux poussoirs droit et gauche des chiffres de 0 à 9 et validation des nombres souhaités, ce qui lui permet de donner des indications horaires exactes, ce système permettant aussi au sujet, comme il sera dit plus loin, de donner éventuellement ses réponses au cours du test de calcul mental;
Pour chaque test, le sujet reçoit un message lui annonçant la nature des épreuves qui vont lui être proposées et éventuellement le type de réponse à donner, par exemple pour un TR répondre de la main droite à la survenue d'un son grave; pour une épreuve de raisonnement logique , mémoriser la proposition logique valide et appuyer sur le curseur droit ou gauche selon que le résultat des combinaisons proposées ensuite est "Vrai" ou "Faux"; pour une épreuve de calcul mental le moyen de donner la réponse, soit sur l'afficheur et les poussoirs soit vocalement; pour une épreuve de barrage de lettres, mémoriser le ou les items affichés et les reconnaître lorsqu'ils apparaissent sur les lignes de l'écran en validant la position correspondante du curseur par appui simultané des poussoirs gauche et droite ; pour une épreuve de scrutage mnésique mémoriser les items tests, et appuyer sur le poussoir droit ou gauche selon que le résultat des combinaisons proposées ensuite est "Vrai" ou "Faux"; pour une épreuve de mémorisation spaciale, mémoriser la figure affichée, appuyer sur le curseur droit ou gauche selon que le résultat des combinaisons proposées ensuite est "Vrai" ou "Faux"; pour une épreuve de force musculaire, appuyer sur le capteur à droite puis à gauche jusqu'à la déviation maximum d' un cuseur sur l'afficheur. Un compteur de temps de résolution égale à 1 ms et au moins un compteur d'évènements sont enclanchés au moment de l'émission du stimulus ou à la fin de la demande de test; ils comptent la durée en ms et s'il y a lieu, le nombre d'évènements objets du test, jusqu'à la fermeture d'un interrupteur en réponse à la requête formulée, ou jusqu'à la durée maximum programmée du test, la durée totale de l'épreuve ne pouvant dépasser une limite programmée, fonction des conditions du test. Dans le cas des mesures de TR l'intervalle entre deux stimuli peut être aléatoire et suit dans ce cas une loi uniforme entre 0.4 et 4 secondes. Les mesures à stocker sont constituées selon le test soit par le temps de réponse, soit par un score, soit par un index d'erreurs soit par un indice de conformité de la réponse à la demande soit par une combinaison de plusieurs de ces valeurs , les dites mesures étant conservées, avec l'indication des jour,heure et minute du début de la séquence de prise de données, dans la seconde mémoire, selon un choix paramétré soit sous la forme de mesures discrètes soit sous une forme statistique consistant alternativement pour chaque série de test en la moyenne et l'écart-type ou en l'histogramme des fréquences avec un nombre de classes paramétré. Pour chaque échelle analogique, et chaque test proposé, un durée maximum est prévue de sorte que le dispositif ne reste pas inutilement actif si le sujet abandonne les épreuves.

Le microprocesseur est ensuite mis en état de basse consommation ou repos (bloc 52) dont il sort périodiquement pour assurer le traitement de l'horloge, le contrôle du bon fonctionnement de l'ensemble du dispositif ( bloc 53) et au moment de l'exécution des tests (bloc 50).

Grâce aux moyens mis en oeuvre dans le cadre de l'invention, on dispose d'un appareil de faible dimension, léger doté de capacités de communication et fonctionnant de façon autonome, qui permet de mesurer, d'enregistrer et d'analyser le temps de réaction suivant différentes modalités et d'apprécier la vigilance d'un sujet selon des séquences pré-établies, et de façon automatique, durant de longues durées et dans toutes conditions environnementales.

Comme il va de soi, et comme il résulte d'ailleurs de ce qui précède, l'invention ne se limite nullement à ceux de ses modes d'application et de réalisation qui ont été plus spécialement envisagés: elle en embrasse au contraire toutes les variantes.

## Revendications

1. Procédé pour la réalisation, de façon autonome, de tests psychométriques et cognitifs et pour la mesure, l'enregistrement et l'analyse de la réponse volontaire d'un sujet humain à ces tests, dans lequel le sujet matérialise sa réponse par la fermeture d'un ou plusieurs interrupteurs(7,8), ces tests étant réalisés par l'intermédiaire d'au moins deux transducteurs optiques (26), d' un transducteur acoustique (24) et d'au moins un transducteur tactile non figuré, d'au moins un dispositif d'affichage (22), sous le contrôle d'une unité de commande (2); lequel procédé comprend les opérations suivantes:
- on procure un dispositif (1) qui comporte lesdits interrupteurs(7,8), lesdits transducteurs(24,26), ledit dispositif d'affichage (22), ladite unité de commande(2), au moins un microprocesseur (4) qui exécute les instructions d'un programme stocké dans une mémoire PROM (18) , lequel dispositif comporte au moins une mémoire RAM (17), une horloge , un dispositif de mesure du temps de réponse , un dispositif de synthèse vocale (23), lequel dispositif comporte un interface bi-directionnel de communication (15),
- on enregistre dans ladite mémoire PROM (18) ledit programme,
- on procure au moins un ordinateur central , et des moyens de communication entre le dit ordinateur central et le dit dispositif,
- on enregistre durant une première phase de communication, dans la dite mémoire RAM (17) les paramètres selon lesquels les tests seront réalisés et selon lesquels les réponses du sujet seront demandées et stockées,
- et dans une phase de fonctionnement autonome et ambulatoire, conformément audit programme et auxdits paramètres on propose successivement tous les tests programmés, et pour chaque test, on fait appel à la procédure d'acquisition de données appropriée, et on conserve les résultats dans ladite mémoire RAM (17), selon un desdits paramètres ou bien sous la forme de mesures discrètes,
- et dans toute phase ultérieure de communication on lit dans la dite mémoire RAM (17), puis on transfère dans ledit ordinateur central (21) lesdites valeurs mesurées,
lequel procédé est caractérisé en ce que
- selon une option programmable, le sujet a la possibilité de matérialiser sa réponse oralement
- ledit dispositif (1) procuré est portable, compact, et comporte un dispositif (9) d'analyse de la parole, une unité (3) de gestion des informations, et une réserve d'énergie électrique,
- la procédure d'acquisition de données appropriée consiste en l'évaluation d'un score, en l'évaluation d'un temps de réponse du sujet, en l'appréciation de la conformité de la réponse aux modalités de la demande, en la mesure d'une résistance électrique, et en la lecture d'un résultat numérique sur le dispositif d'affichage,
- les résultats sont conservés selon une option programmable soit sous forme brute, soit sous une forme statistique prédéfinie consistant pour chaque épreuve, en la moyenne, l'écart-type, et les histogrammes de fréquences des performances et de l'index de conformité.

2. Procédé selon la revendication 1 caractérisé en ce que dans la dite phase de fonctionnement autonome
- on stocke dans la dite mémoire (17) la mesure temporelle entre le début d'un test et la fermeture d'un interrupteur par le sujet,
- on détermine si l'interrupteur activé correspond à la modalité demandée au sujet, et on associe à la mesure proprement dite, dans ladite mémoire (17), une information je conformité,

3. Procédé selon les revendications 1 et 2 caractérisé en ce qu'il permet de mesurer le temps de réaction simple c'est à dire le temps de réponse à un stimulus déterminé annoncé avec la modalité de réponse associée, et qu'il permet de mesurer aussi le temps de réaction de choix, c'est à dire le temps de réponse du sujet à un stimulus visuel ou auditif qui lui est fourni selon un mode c'est à dire une couleur ou une fréquence, exclusivement associé à un moyen de réponse à la disposition du sujet, l' indication de cette association n'étant pas fournie durant le test, mais devant être mémorisée par le sujet au préalable au cours d' épreuves d'apprentissage et d' entrainement, lesdits temps de réponse étant évalués en millisecondes, et codés de façon à conserver une information sur la qualité de la réponse, c'est à dire l'indication du moyen de réponse utilisé, et de l'anticipation ou non de la réponse,

4. Procédé selon les revendications 1 à 3 caractérisé en ce qu'il permet d'effectuer des tests de calcul mental, en proposant au sujet par l'intermédiaire de l' afficheur (22) ou du dispositif de synthèse vocale (23) des séries d' opérations arithmétiques, les nombres et les opérateurs étant tirés au hasard sous des contraintes programmables portant sur le nombre de chiffres des opérandes, le type des opérateurs utilisés, et le signe et la grandeur des résultats,

5. Procédé selon les revendications 1 à 4 caractérisé en ce qu'il permet d'effectuer des tests de barrage de lettres durant lesquels le sujet doit mémoriser un ou plusieurs signes qui peuvent être des lettres de l'alphabet, examiner ensuite un ensemble de lignes de signes sur un ou plusieurs écrans présentés successivement, reconnaitre les signes mémorisés et valider cette reconnaissance à l'aide d'un curseur se déplaçant à droite et à gauche, le temps de balayage de l'écran avec le curseur, le nombre de signes validés, le nombre exact de signes à reconnaitre, le nombre d'erreurs, et le nombre de signes validés en double permettant d'évaluer la qualité de la réponse,

6. Procédé selon les revendications 1 à 5 caractérisé en ce qu'il permet de réaliser des tests de scrutage mnésique durant lesquels le sujet doit mémoriser un ou plusieurs mots, examiner ensuite un ou plusieurs ensembles de mots, désignés sous le terme de cibles, pendant un temps donné, et reconnaître lorsque la cible disparaît si elle contenait le ou les mots mémorisés, le test étant réalisé soit au moyen de l'afficheur (22), soit au moyen du dispositif de synthèse vocale (23), par tirage au sort de mots pris dans une liste composée de mots contenus dans les mémoires ROM (18) et RAM (17),

7. Procédé selon les revendications 1 à 6 caractérisé en ce qu'il permet de réaliser des tests de raisonnement logique durant lesquels le sujet doit mémoriser une proposition logique dont les objets et les opérateurs sont tirés au sort dans deux listes comportant au moins les lettres de l'alphabet pour ce qui concerne les objets et au moins les opérateurs logiques ET, OU, NON, au moins les opérateurs relationnels >, <, =, <>, et au moins les prédicats "PRECEDE", "SUIT", "AVANT", "APRES", pour ce qui concerne les opérateurs, une nouvelle proposition logique étant ensuite soumise au sujet qui doit en apprécier la conformité à la proposition logique mémorisée, en répondant par "OUI" ou "NON par le biais des poussoirs ou en signifiant oralement sa réponse au dispositif d'analyse de la parole,

8. Procédé selon les revendications 1 a 7 caractérisé en ce qu'il permet de réaliser des tests de mémorisation spaciale durant lesquels le sujet doit mémoriser une figure géométrique composée a partir d'éléments tirés au sort dans une liste composée d'éléments fixes et d'éléments transmis durant la phase de programmation du dispositif, regarder ensuite une figure construite de la même manière, ayant éventuellement subi une rotation à gauche ou à droite, mais présentant éventuellement des différences, et détecter si l'enveloppe de la deuxième figure est la même que celle de la première,

9. Procédé selon les revendications 1 a 8 caractérisé en ce qu'il permet de réaliser des tests de pistage instable c'est à dire de proposer au sujet de maintenir pendant un temps déterminé un symbole de pointage au plus près d'un symbole crée sur l'afficheur, désigné comme cible et animé de mouvements aléatoires, sous la contrainte que la correction de la position de ce symbole engendre une deviation de la cible, la qualité du score étant mesurée par la distance moyenne entre le symbole et la cible, et le test étant réalisé au moyen d'un afficheur graphique et d'un film résistif de détection de position permettant de connaître la position du point où le sujet appuie un stylet ,

10. Procédé selon les revendications 1 à 9 caractérisé en ce qu'il permet de réaliser des tests de tâche double, c'est à dire de proposer au sujet simultanément et selon des directives transmises durant la phase de programmation, simultanément deux tests compatibles dans leur accomplissement,

11. Procédé selon les revendications 1 à 10 caractérisé en ce qu'il permet la quantification de paramètres subjectifs psychiques et physiques du sujet, comme la qualité de son humeur et de son sommeil et l'intensité de sa douleur, par le biais d' échelles d'auto- estimation, en demandant au sujet de positionner un index sur un segment de droite symbolisant l'amplitude du paramètre entre son minimum et son maximum, cet index pouvant être un curseur se déplaçant sur un afficheur (22) ou une pointe rigide appuyée par le sujet en un point d'un film résistif de détection de position (14),

12. Procédé selon les revendications 1 à 11 caractérisé en ce qu'il permet de réaliser des tests de force musculaire par le bials d' un film résistif de détection de force (12) placé sur la face supérieure du boitier, sur lequel le sujet appuie avec le pouce en faisant réaction sur la face inférieure du boîtier avec les autres doigts,

13. Procédé selon les revendications 1 à 12 caractérisé en ce qu'il permet au sujet de communiquer des nombres au dispositif, par défilement et validation de chiffres sur l' afficheur (22), au moyen de poussoirs (7,8),

14. Procédé selon les revendications 1 à 13 caractérisé en ce qu'il permet au sujet de communiquer oralement des réponses au dispositif, par le biais d'un microphone et d'un dispositif d'analyse de la parole.

15. Dispositif pour la réalisation de tests psychométriques et la délivrance de stimuli, de façon autonome, au moyen de transducteurs optiques et acoustique (24,26), pour effectuer la mesure et l'enregistrement de la réponse volontaire d'un sujet humain auquel ces tests et stimuli sont destinés, réaction matérialisée par la fermeture d'un ou plusieurs interrupteurs à contact temporaire (7,8), lequel dispositif comporte un dispositif électronique de synthèse vocale (23) fournissant au sujet des messages vocaux, lequel dispositif comporte à l'intérieur d'un boitier au moins un circuit électronique comprenant un microprocesseur (4), une mémoire ROM (18), une mémoire RAM (17), une horloge, au moins deux interrupteurs (7), lesdits transducteurs (24,26), ledit dispositif d'affichage (22) et ledit dispositif de synthèse vocale (23) associé à une mémoire PROM non figurée, un interface bi-directionnel de communication (15), des moyens d'avertissement acoustiques et visuels qui peuvent être actionnés par le dit microprocesseur (4), caractérisé en ce qu'il comporte un dispositif (22) d'affichage à cristaux liquides, un dispositif (9) électronique d'analyse de la parole, un microphone (10), au moins une prise pour interrupteur externe (8), une réserve d'énergie électrique, et en ce que ledit dispositif a un volume et un poids tels qu'il est portable, et en ce que lesdits moyens d'avertissement acoustiques et visuels peuvent être actionnés, en cas de défaillance dudit microprocesseur, par un circuit chien de garde (28), de sorte que le sujet peut être informé d'une anomalie détectée dans le fonctionnement dudit dispositif.

## Claims

1. Method for the realization, in autonomous manner, of psychometric and cognitive tests for measure, recording and analysis of the voluntary response of a human subject to these tests, in which one the subject materialise his response by closing of one or several switches (7,8), these tests being realised by the means of at least two optical transducers (26), of one acoustic transducer (24) and at least a non figured tactile transducer, at least a display device (22), under the control of a control unit (2), that involves the following operations:
- a device (1) is supplied, consisting of said switches(7,8), said transducers(24,26), said display device (22), said control unity (2), at least a microprocessor (4) that executes the instructions of a stocked program in a PROM memory (18), which one device includes at least a RAM memory (17), a clock, a measure time device response, a device voice synthesis (23), which one device includes a bi-directional interface of communication (15),
- the said program is recorded in the said memory PROM (18),
- at least a central computer, and means of communication between the said central computer and the said device are supplied,
- during a first communication phase are registered in the said RAM memory (17) the parameters according to which the tests will be realised and the responses of the subject will be required and stocked,
- and in an autonomous and ambulatory working phase, in conformity with said programs and said parameters successively all the programmed tests are proposed, and for each test, the adapted data collection procedure is applied, and the results are stocked in said RAM memory (17), according to said parameters or as discrete measures,
- and in all later phase of communication the said measured values are read in said RAM memory (17), then transferred in said central computer (21),
which one method is characterised in what
- according to a programmable option, the subject has the possibility to orally materialise his response
- the said get device (1) is portable, compact, and includes a device (9) of speech analysis, an information management unit (3), and a reserve of electric energy,
- the data acquisition procedure consists in the evaluation of a score, in the evaluation of a response time of a subject, in the estimating of the accordance of the response with the modalities of the request, in the measure of an electric resistance, and in the reading of a result on the display device,
- the results are stocked according to a programmable option either as raw data or under predefined statistical form, consisting for each test in the average, the standard error, and the frequency histograms of performances and accordance index.

2. Method according to claim 1 characterised by the fact that in the said autonomous functioning phase
- the temporal measure between the beginning of the test and the closing of the switch by the subject is stocked in the said memory (17),
- it is determined if the activated switch by the subject corresponds to the required modality, and an accordance information is associated to the measure stocked in said memory (17),

3. Method according to claims 1 and 2 characterised by the fact that it gives the possibility to measure the simple reaction time this is to say the response time to a determined stimulus, announced with the modality of the associated response, and by the fact that it gives the possibility to also measure the reaction time with choice, this is to say the response time of the subject to a visual or audio stimulus that is delivered according to a modality, this is to say a colour or a frequency, exclusively associated with a mean of response at the disposal of the subject, the indication of this association being not given during the test, but having to be memorised by the subject during a previous learning and training phases, the said response time being evaluated in milliseconds, and coded so as to preserve an information on the quality of the response, this is to say the indication of the used means of response and of anticipated or not anticipated response,

4. Method according to the claims 1 to 3 characterised one in what it gives the possibility to realise tests of mental calculation, which consists in proposing to the subject, by means of the display (22) or the voice synthesis device (23), some series of arithmetic operations, the operands and the operators being drawn at random under programmable constraints concerning the number of digits for operands, the nature of used operators, the sign and the magnitude of results,

5. Method according to the claims 1 to 4 characterised one in what it gives the possibility to realise letters cancellation tests during which these the subject has memorised one or several signs that can be letters of the alphabet, examine next a group of lines of signs on one or several screens successively presented, recognise the memorised signs and validate this recognition by the means of a cursor moving itself to right and to left, the screen scanning time with the cursor, the number of validated signs, the exact number of signs to recognise, the number of errors, and the number of double validated signs allowing to evaluate the quality of the response,

6. Method according to the claims 1 to 5 characterised one in what it allows to realise mnesic scanning tests during which these the subject has to memorise one or several words, next examine one or several groups of words, designated under the term of targets, during a given time, and recognise when the target disappears if it contained the one or several memorised words, the test being realised by means of the display (22), or by means of the voice synthesis device (23), by drawing at random of words take in a list composed by words contained in the ROM (18) and RAM (17) memories,

7. Method according to the claims 1 to 6 characterised one in what it allows to realise logical reasoning tests during which the subject has to memorise a logical proposition such as the objects and the operators are drawn at random in two lists consisting of at least the letters of the alphabet for the objects and at least the logical operators AND, OR, NOT, at least the relational operators >, <, =, <>, and at least predicates "PRECEDE", "FOLLOWS", "BEFORE", "AFTER", for the operators, a new logical proposition next being submitted to the subject that has to assess its accordance to the memorised logical proposition, and give a response by "YES" or "NOT" manually by means of the push buttons or orally by means of the speech analysis device,

8. Method according to the claims 1 to 7 characterized in what it allows to realise spatial memorization tests during which the subject has to memorise a geometric figure composed from drawn at random elements in a list composed by fixed elements and by elements transmitted during the programming device phase, next the subject looks a figure built of the same manner, with eventually a rotation to the left or to right, but eventually presenting some differences, and the subject detects if the envelope of the second figure is the same that the first one,

9. Method according to the claims 1 to 8 characterised one in what it allows to realise unstable tracking tests this is to say to propose on the subject of maintain during a determined time a pointing symbol the more close to a symbol created on the display, designated as target and animated of random movements, under the constraint than the correction of the position of the pointing symbol produces a deviation of the target, the quality of the score being measured by the averaged distance between the symbol and the target, and the test being realised by means of a graphic display and of a resistive film of position detection to know the position of the point where the subject supports a probe,

10. Method according to the claims 1 to 9 characterised one in what it allows to realise double task tests, this is to say to propose to the subject simultaneously and according to transmitted directives during the programming phase, simultaneously two tests compatible in their accomplishment,

11. Method according to the claims 1 to 10 characterised one in what it allows the quantification of subjective psychic and physical parameters of a subject, such as the quality of his mood and of his sleep and the intensity of his pain, by means of auto-estimation scales, requiring on the subject of point an index on a straight line segment representing the amplitude of the parameter between his minimum and his maximum, this index being a cursor moving on a display (22) or a rigid point pressed by the subject in a point of a resistive film for position detection (14),

12. Method according to the claims 1 to 11 characterised one in what it allows to realise tests of muscular force by the means of a resistive film for force detection (12) placed on the superior face of the case, on which the subject presses with the thumb while doing reaction on the inferior face of the case with the other fingers,

13. Method according to the claims 1 to 12 characterised one in what it allows on the subject of communicate numbers to the device, by scrolling and validation of digits on display (22), by means of push buttons (7,8),

14. Method according to the claims 1 to 13 characterised one in what it allows on the subject of communicate orally responses to the device, by the means of a microphone and of a speech analysis device,

15. Device for the realisation of psychometrics tests and the stimuli delivery, of autonomous manner, by means of optical and acoustic transducers (24,26), to effectuate the measure and the recording of the voluntary response of a human subject to which one these tests and stimuli are destined, reaction materialised by the closing of one or several switches with temporary contact (7,8), which one device comprises an electronic device of voice synthesis (23) supplying to the subject the vocal messages, which one device comprises inside a case at least an electronic circuit including a microprocessor(4), a memory ROM(18), a memory RAM(17), a clock, at least two switches (7), said transducers (24,26), said display device (22) and said device of voice synthesis (23) associated with a memory non figured PROM, a bi-directional interface of communication (15), means of acoustic and visual warning that can be activated by the says microprocessor(4), characterised in what it includes a device (22) of display to crystals liquidate, a device (9) electronic of speech analysis, a microphone(10), at least a plug for external switch (8), a reserve of electric energy, and in what said device has a volume and a weight such that it is portable, and in what said means of acoustic and visual warning can be activated, in case of failure of the said microprocessor, by a watch dog (28) circuit, so that the subject can be informed of a abnormality detected in the said device working.

## Patentansprüche

1. Vorrichtung zur Herstellung, auf unabhängige Weise, psychometrischer und kognitiver Teste und zur Messung, Aufzeichnung und Analyse der freiwilligen Antwort einer Versuchsperson auf diese Teste, wo die Versuchsperson beim Abschalten eines oder mehreren Schaltern antwortet (7,8), wobei diese Teste durch die Vermittlung wenigstens zwei optischer Meßwandler (26), eines akustischen Meßwandlers (24) und wenigstens eines nicht dargestellten Tastmeßwandlers, wenigstens einer Anzeigetafel unter der Kontrolle einer Steuereinheit (2) gemacht werden; welche Vorrichtung folgendermaßen arbeitet :
- eine Einrichtung (1) wird verschaffen, die aus den genannten Schaltern (7,8), aus den genannten Meßwandlern (24,26), aus der genannten Anzeigetafel (22), aus der genannten Steuereinheit (2), aus wenigstens einem Mikroprozeßor (4) besteht, der die Anweisungen eines in einem PROM-Speicher (18) aufbewahrten Programms ausführt, welche Einrichtung aus wenigstens einem RAM-Speicher (17), aus einer Uhr, aus einem Meßgerät, das die Antwortszeit mißt, aus einer Einrichtung für elektronisch erzeugte Sprache (23) besteht, welche Einrichtung aus einem Interface zur beidseitigen Datenübermittlung (15) besteht,
- das genannte Programm wird zum genannten PROM-Speicher (18) übertragen,
- es werden wenigstens ein Hauptrechner und Übertragungsmedia zwischen dem genannten Hauptrechner und der genannten Einrichtung verschaffen,
- in einer ersten Übertragungsphase werden die Parameter zum genannten RAM-Speicher (17) übertragen, die es bestimmen, wie die Teste gemacht werden, wie die Versuchsperson zu den Antworten aufgefordert wird und wie diese Antworten gespeichert werden,
- und in einer ambulanten und unabhängigen Betriebsphase werden - dem genannten Programm und den genannten Parametern gemäß - alle programmierte Teste nacheinander gemacht und für jeden Test wird der geeignete Datenerfassungsprozeß hervorgerufen und werden die Werte - entweder einem der genannten Parameter gemäß oder als diskrete Maße - zum genannten RAM-Speicher (17) übertragen,
- und in jeder späteren Übertragungsphase wird im genannten RAM-Speicher (17) gelesen, dann werden die genannten gemessenen Werte zum genannten Hauptrechner (21) übertragen,
Vorrichtung dadurch gekennzeichnet, daß
- die Versuchsperson die Möglickeit hat, nach einer programmierbaren Wahl mündlich zu antworten,
- die genannte Einrichtung (1) tragbar, kompakt ist, und aus einer Sprachanalyse-Einrichtung (9), aus einer Datenverwaltungseinheit (3), und aus einer Reserve elektrischer Energie besteht,
- der Erfassungsprozeß geeigneter Daten aus einer Abschätzung der Ergebnisse, aus einer Abschätzung der Antwortszeit, aus der Abschätzung, in welchem Maße die Antwort den Aufforderungsmodalitäten entspricht, aus der Messung eines elektrischen Widerstandes und aus dem Lesen eines Digitalergebnisses auf der Anzeigetafel besteht,
- die Ergebnisse entweder als unbearbeitete Daten oder als vorher bestimmte Statistik einer programmierbaren Option gemäß behalten werden, welche Statistik für jeden Test aus dem Mittelwert, aus der Musterabweichung, aus den Frequenzhistogranmen der Leistungen und Frequenzhistogrammen des Übereinstimmungsindexes besteht.

2. Vorrichtung nach Anspruch 1, dadurch gekennzeichnet, daß in der genannten unabhängigen Betriebsphase
- die Zeit zwischen dem Anfang eines Tests und dem Abschalten eines Schalters durch die Versuchsperson gemessen wird und dieser Zeitmaß zum genannten Speicher (17) übertragen wird,
- festgelegt wird, ob der aktivierte Schalter der von der Versuchsperson aufgeforderten Modalität entspricht, und daß im genannten Speicher (17) eine Übereinstimmungsinformation dem eigentlichen Maße assoziiert wird,

3. Vorrichtung nach Ansprüche 1 und 2, dadurch gekennzeichnet, daß sie es erlaubt, die einfache Reaktionszeit, d. h. die mit der assoziierten Antwortsmodalität angekündigte Antwortszeit nach einem bestimmten Reiz zu messen, und daß sie es auch erlaubt, die Wahlreaktionszeit zu messen, d.h. die Antwortszeit der Versuchsperson nach einem ihr einer Anweisung gemäß gegebenen visuellen oder auditiven Reiz, d.h. einer Farbe oder einer Frequenz, welche Anweisung mit einem zur Verfügung der Versuchsperson stehenden Antwortsmittel ausschliesslich assoziiert ist, wobei die Anweisung auf diese Assoziation während des Tests nicht gegeben wird, da sich die Versuchsperson dieser Anweisung durch Erlernen-und Übungsteste vor dem Test einprägen sollte, und wobei die genannten Antwortszeiten in Millisekunden gemessen werden und derart verschlüsselt werden, daß eine Information über die Qualität der Antwort bewahrt wird, d.h. die Anweisung auf das angewandte Antwortsmittel und auf einen - oder auf keinen - Vorgriff der Antwort,

4. Vorrichtung nach einem der Ansprüche 1 bis 3, dadurch gekennzeichnet, daß sie es erlaubt, Kopfrechnenteste zu machen, wobei durch die Anzeigetafel (22) oder durch die Einrichtung für elektronisch erzeugte Sprache (23) der Versuchsperson Rechenoperationsreihen gegeben werden, wo die Zahlen und die Operatoren unter programmierbaren Bedingungen durch Auslosen gezogen werden, welche Bedingungen die Zahl der Ziffer der Operanden, die Art der angewandten Operatoren und das Zeichen und die Größe der Ergebnisse betreffen,

5. Vorrichtung nach einem der Ansprüche 1 bis 4, dadurch gekennzeichnet, daß sie es erlaubt, Buchstabendurchstreichenteste zu machen, wobei die Versuchsperson sich einem oder mehreren Zeichen, die Alphabetbuchstaben sein können, einprägen soll, auf einem oder mehreren nacheinander gezeigten Bildschirmen sich einer Zeichenreiheneinheit genau ansehen soll, die eingeprägten Zeichen erkennen und dieses Erkennen mit Hilfe eines sich rechts und links bewegenden Cursors bestätigen soll, wobei die Bewegungszeit des Cursors auf dem Bildschirm, die Zahl der bestätigten Zeichen, die genaue Zahl der zu erkennenden Zeichen, die Fehlerzahl und die Zahl der doppeltbestätigten Zeichen es erlauben, die Qualität der Antwort abzuschätzen,

6. Vorrichtung nach einem der Ansprüche 1 bis 5, dadurch gekennzeichnet, daß sie es erlaubt, Gedächtnisforschungsteste zu machen, während welcher die Versuchsperson sich einem oder mehreren Wörtern einprägen soll, sich nachher während einer gewissen Zeit eine oder mehrere als Ziele bezeichnete Wörtereinheiten genau ansehen soll, und beim Verschwinden des Ziels erkennen soll, ob das Ziel das eingeprägte Wort - oder die eingeprägten Wörter - enthielt, wobei der Test mit Hilfe entweder der Anzeigetafel (22) oder der Einrichtung für elektronisch erzeugte Sprache (23) durch Auslosen von Wörtern gemacht wird, die aus einer Liste stammen, die aus in den ROM (18)- und RAM (19)-Speichern enthaltenen Wörtern besteht,

7. Vorrichtung nach einem der Ansprüche 1 bis 6, dadurch gekennzeichnet, daß sie es erlaubt, Logikteste zu machen, während welcher die Versuchsperson sich einem logischen Vorschlag einprägen soll, dessen Objekte und Operatoren auf zwei Listen aufgelost werden, die aus wenigstens den Alphabetbuchstaben - was die Objekte betrifft - und aus wenigstens den logischen Operatoren UND, ODER, NEIN, aus wenigstens den Relationsoperatoren >, <, =, < >, und aus wenigstens den Prädikaten "STEHT VOR", "STEHT NACH", "VORHER", "NACHHER" - was die Operatoren betrifft - bestehen, wobei der Versuchsperson ein neuer logischer Vorschlag danach gemacht wird und wobei die Person die Übereinstimmung dieses Vorschlags mit dem eingeprägten Vorschlag abschätzen soll, indem sie darauf durch Knopfdrücken entweder mit "JA" oder "NEIN" antwortet oder mit Hilfe der Sprachanalyse-Einrichtung mündlich antwortet,

8. Vorrichtung nach einem der Ansprüche 1 bis 7, dadurch gekennzeichnet, daß sie es erlaubt, Raumeinprägenteste zu machen, während welcher sich die Versuchsperson einer geometrischen Figur einprägen soll, die aus Elementen besteht, die auf einer Liste aufgelost werden, die aus bestimmten und aus den während der Einrichtungprogrammierungsphase gespeicherten Elementen besteht, sich dann einer auf die gleiche Weise gezeichnete Figur ansehen soll, die eine nach links oder nach rechts Rotationsbewegung eventuell erfahren hat, und schließlich aufdecken soll, ob die einhüllende Fläche der zweiten Figur die gleiche ist wie die der ersten Figur,

9. Vorrichtung nach einem der Ansprüche 1 bis 8, dadurch gekennzeichnet, das sie es erlaubt, Labilverfolgenteste zu machen, d.h. daß die Versuchsperson darum gebeten wird, während einer gewissen Zeit ein Zeigsymbol so nahe wie möglich an einem auf der Anzeigetafel gezeigten Symbol zu halten, das als Ziel genannt wird und das sich zufällig bewegt, dieses unter der Bedingung, daß die Korrektur der Position dieses Symbols ein Ablenken des Ziels verursacht, wobei die Qualität des Ergebnisses durch die Entfernung zwischen dem Symbol und dem Ziel gemessen wird, und wobei der Test mit Hilfe einer grafischen Anzeigetafel und eines positionsempfindlichen Films gemacht wird, der es erlaubt, die Position des Punktes zu kennen, wo die Versuchsperson eine Anzeigenadel drückt,

10. Vorrichtung nach einem der Ansprüche 1 bis 9, dadurch gekennzeichnet, daß sie es erlaubt, Doppeltarbeitteste zu machen, d.h., daß die Versuchsperson darum gebeten wird, simultan - und während der Programmierungsphase übermittelten Weisungen gemäß - zwei in ihrer Ausführung vereinbare Teste gleichzeitig zu machen,

11. Vorrichtung nach einem der Ansprüche 1 bis 10, dadurch gekennzeichnet, daß sie es erlaubt, psychische und physische subjektive Parameter der Versuchsperson mit Hilfe Selbstschätzungsskalen quantitativ zu erfassen, wie die Qualität ihrer Stimmung und ihres Schlafes, und die Intensität ihres Schmerzes, wobei die Versuchsperson darum gebeten wird, einen Zeiger auf einem Geradesegment zu positionieren, das für die Amplitude des Parameters zwischen Höchst-und Tiefstwerten steht, welcher Zeiger ein sich auf einer Anzeigetafel (22) bewegender Cursor oder eine steife Spitze sein kann, die die Versuchsperson auf einem Punkt eines positionsempfindlichen Films (14) drückt,

12. Vorrichtung nach einem der Ansprüche 1 bis 11, dadurch gekennzeichnet, daß sie es erlaubt, Muskelkraftteste mit Hilfe eines kraftempfindlichen Films (12) zu machen, der auf der oberen Seite des Gehäuses gelegt wird, auf welchem die Versuchsperson mit ihrem Daumen drückt, während sie mit den anderen Fingern auf der unteren Seite des Gehäuses drückt,

13. Vorrichtung nach einem der Ansprüche 1 bis 12, dadurch gekennzeichnet, daß sie es der Versuchsperson erlaubt, mit Hilfe von Knöpfen (7,8) durch Abläufen und Bestätigung von Ziffern auf der Anzeigetafel (22) der Einrichtung Zahlen zu übertragen,

14. Vorrichtung nach einem der Ansprüche 1 bis 13, dadurch gekennzeichnet, daß sie es der Versuchsperson erlaubt, mit Hilfe eines Mikrofons und mit Hilfe einer Sprachenanalyse-Einrichtung der Einrichtung mündliche Antworten zu geben,

15. Eigenständige Einrichtung zur Herstellung psychometrischer Teste und zur Reizerzeugung mit Hilfe optischer und akustischer Meßwandler (24,26), um die freiwillige Antwort einer menschlischen Versuchsperson zu messen und zu speichern, für welche diese Teste und Reize bestimmt sind, wobei die Reaktion der Versuchsperson durch das Abschalten eines oder mehrern Schaltern mit zeitweiser Berührung (7,8) gekennzeichnet wird, welche Einrichtung aus einer Einrichtung für elektronisch erzeugte Sprache (23) besteht, die der Versuchsperson Stimmmeldungen sendet, welche Einrichtung aus wenigstens einem elektronischen Stromkreis innerhalb eines Gehäuses besteht, der aus einem Mikroprozessor (4), aus einem ROM-Speicher (18), aus einen RAM-Speicher (17), aus einer Uhr, aus wenigstens zwei Schaltern (7), aus den genannten Meßwandlern (24,26), aus der genannten Anzeigetafel (22) und aus der genannten mit einem nicht dargestellten PROM-Speicher assoziierten Einrichtung für elektronisch erzeugte Sprache (23), aus einem Interface zur beidseitigen Datenübermittlung (15), aus akustischen und visuellen Warnsignalen, die durch den genannten Mikroprozessor (4) aktiviert werden können, besteht, dadurch gekennzeichnet, daß sie aus einer Flüssigkristallanzeige-Einrichtung (22), aus einer elektronischen Sprachanalyse-Einrichtung (9), aus einem Mikrofon (10), aus wenigstens einer Steckdose für einen äußeren Schalter (8), aus einer elektrischen Energiereserve besteht, und dadurch gekennzeichnet, daß die genannten akustischen und visuellen Warnsignale im Falle eines Defekts des genannten Mikroprozessors durch einen Überwachungsstromkreis (28) aktiviert werden können, so daß die Versuchsperson über einen im Betrieb der genannten Einrichtung aufgedeckten Defekt informiert werden kann.
